# EUROPEAN PATENT SPECIFICATION

(11) **EP 2 813 497 B1**
(45) Date of publication and mention of the grant of the patent: **28.08.2019**
(21) Application number: 13003038.0
(22) Date of filing: 13.06.2013
(51) Int. Cl.: C07D 317/36, C07D 405/12, A61K 6/087

(54) **Dental composition**
Dentale Zusammensetzung
Composition dentaire

(43) Date of publication of application: 17.12.2014
(73) Proprietor: Dentsply DeTrey GmbH, 78467 Konstanz (DE)
(72) Inventor: Dr. Klee, Joachim, 78315 Radolfzell (DE); Dr. Retzmann, Nils, 40215 Düsseldorf (DE); Szillat, Florian, 40474 Düsseldorf (DE); Prof. Dr. Ritter, Helmut, 42111 Wuppertal (DE)
(74) Representative: Dietz, Mirko

(56) References cited:
- EP-A1- 0 779 290
- WO-A2-2007/038764

## Description

### Field of the Invention

The present invention relates to a dental composition selected from a root canal sealing composition and a pulp capping composition, which comprises a compound polymerizable in a step-growth polymerization reaction based on at least two optionally substituted 1, 3-dioxolane-2-one or 2,3-carbonatopropyl groups linked to or connected by one or more organic groups.

A compound polymerizable with a monomer having at least two groups selected from primary and secondary amino groups and thiol groups in a step-growth polymerization reaction based on at least two optionally substituted 1, 3-dioxolane-2-one or 2,3-carbonatopropyl groups linked to or connected by one or more organic groups is disclosed, and the present invention relates also to a process for the production of the compound.

Furthermore, the present invention relates to a use of the polymerizable compound for the preparation of a dental composition selected from a root canal sealing composition and a pulp capping composition.

The polymerizable compound contained in the dental composition according to the present invention may be used for avoiding bisphenol-A based components such a bisphenol A diglycidyl ether in a root canal sealing composition or pulp capping composition due to the favorable mechanical properties of the cured compositions and the low viscosity of the uncured compositions, as well as the small dimensional changes of the compositions upon curing.

### Background of the Invention

Dental compositions are desired to approach natural tooth structure with regard to strength and appearance. Accordingly, a great effort is documented by the prior art directed to the development of dental compositions having improved properties with regard to physical properties, biocompatibility, aesthetics and handling properties.

Dental compositions selected from a root canal sealing composition and a pulp capping composition are subject to additional requirements in that the cured product is required to have a high radioopacity and in that the composition may only be cured in the absence of light. Moreover, it is desireable that the composition adheres to the wall of the root canal in order to further improve the tight sealing of the dental root canal.

Accordingly, a root canal sealing composition or pulp capping composition contains radioopaque particulate fillers, which is dispersed in a curable matrix . The dispersion of radioopaque particulate fillers gives rise to a stability problem of the dispersions due to the high density of the filler and the low viscosity of the curable matrix.

Moreover, in order to be able to cure a root canal sealing composition or pulp capping composition in the absence of light, the composition is cured by a thermal curing mechanism which may involve step growth polymerizing epoxide precursor compounds such as bisphenol A diglycidylether. Bisphenol A diglycidyl ether provide an excellent combination of properties for the purpose of a dental compositions. Specifically, favorable mechanical properties of the cured compositions while the viscosity of the uncured compositions may be adjusted to be comparably low, and a low shrinkage of the compositions upon curing are reasons for the widespread use of bisphenol A based materials in the dental field.

However, epoxide precursors may be irritants and are problematic with regard to carcinogenicity and mutagenicity.

Moreover, bisphenol A is a known endocrine disrupter which can mimic estrogen and may lead to negative health effects. More specifically, bisphenol A mimics the structure and function of the hormone estradiol with the ability to bind to and activate the same estrogen receptor as the natural hormone. Based on the functional relevance of bisphenol-A it is considered that bisphenol-A might contribute to the development of breast cancer. Accordingly, regulatory bodies might determine safety levels of bisphenol-A for humans so that the use of bisphenol A based materials containing bisphenol A in a dental composition cannot be continued in the future.

WO 2007/038764 discloses an epoxy based oil free root canal sealer

### Summary of the Invention

Accordingly, it is the problem of the present invention to provide a dental composition selected from a root canal sealing composition and a pulp capping composition, having properties including physical properties of the cured composition, dispersion stability and handling properties of the uncured composition, and biocompatibility, which are at least on the level of corresponding bisphenol A based materials, while the bisphenol-A component is replaced by a new type of polymerizable component. Moreover, the compositions should show adhesion to the wall of a dental root canal in order to further improve the tight sealing of a root canal.

It is a further problem of the present invention to provide a process for preparing the dental composition selected from a root canal sealing composition and a pulp capping composition which is cost efficient and simple and which may be carried out on a scale which is industrially relevant for the preparation of a dental composition.

Finally, it is the problem of the present invention to provide, a polymerizable compound obtainable in the process of the present invention as well as a use of the polymerizable compound for the preparation of a dental composition.

According to a first aspect, the present invention provides a dental composition selected from a root canal sealing composition and a pulp capping composition, which comprises:
(a) a radioopaque particulate filler;
(b) a monomer having at least two groups selected from primary and secondary amino groups and thiol groups; and
(c) a compound polymerizable with the monomer (b) in a step-growth polymerization reaction based on at least two groups selected from the following formula (A) and (B) attached to or connected by one or more organic groups: 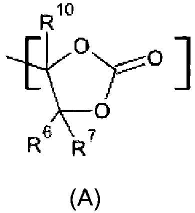 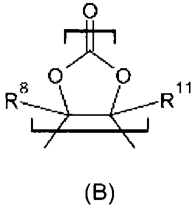 R⁶, R⁷ R⁸, R¹⁰ and R¹¹ which are independent from each other, represent a hydrogen atom or a C₁₋₆ alkyl group.

According to a second aspect, a compound polymerizable with a monomer is disclosed, which monomer has at least two groups selected from primary and secondary amino groups and thiol groups in a step-growth polymerization reaction based on at least two groups selected from the following formula (A) and (B) attached to or connected by one or more organic groups: 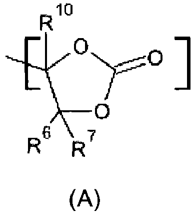 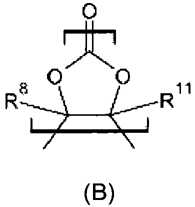 R⁶, R⁷ R⁸, R¹⁰ and R¹¹ which are independent from each other, represent a hydrogen atom or a C₁₋₆ alkyl group
which compound is obtainable
(c1) by reacting
   (i) at least (n+1) moles of one or more compounds of the following formula (I): 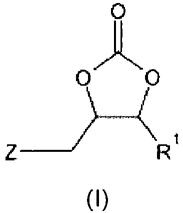 wherein
      Z is a hydroxyl group, a thiol group, or a group -NHR^{∘}, wherein R^{∘} is a C₁₋₆ alkyl group,
      R¹ is a hydrogen atom or a C₁₋₆ alkyl group,
   (ii) with one mole of a compound of the following formula (II): 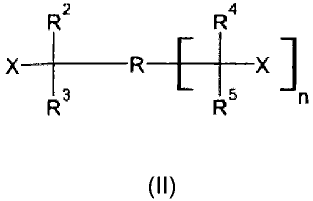 wherein
      R² and R³ which are independent from each other, represent a hydrogen atom or a C₁₋₆ alkyl group,
      the R⁴, and R⁵, which may be the same or different and which are independent from each other, represent a hydrogen atom or a C₁₋₆ alkyl group,
      the X, which may be the same or different, represent a group selected from a carboxylic acid halide group, an isocyanate group, and a leaving group,
      R is an (n+1)-valent organic group, and
      n is an integer of from 1 to 3; or
(c2) by reacting carbon dioxide with the an epoxy resin of the following formula (III): 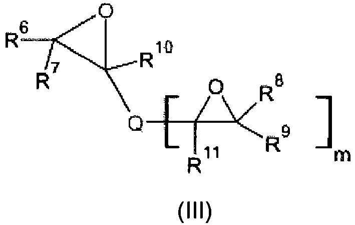 wherein
   Q is an (m+1)-valent organic group,
   R⁶ and R⁷ which are independent from each other, represent a hydrogen atom or a C₁₋₆ alkyl group,
   the R⁸, and R⁹, which may be the same or different and which are independent from each other, represent a hydrogen atom or a C₁₋₆ alkyl group,
   m is an integer of from 1 to 3; or
(c3) by reacting phosgene with the a polyol of the following formula (IV) 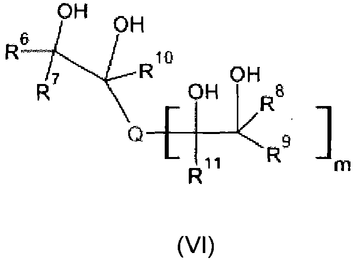 wherein Q, R⁶, R⁷, the R⁸, the R⁹, R¹⁰, the R¹¹, and m are independently as defined for formula (III).

According to a third aspect, the present invention provides a process for preparing the polymerizable compound according to the second aspect, which comprises step (c1), (c2) and/or (c3) as defined in the second aspect of the present invention.

According to a fourth aspect, the present invention provides a use of the polymerizable compound according the second aspect of the present invention for the preparation of a dental composition selected from a root canal sealing composition and a pulp capping composition.

The present invention is based on the recognition that a polymerizable compound having at least two optionally substituted 1, 3-dioxolane-2-one or 2,3-carbonatopropyl groups linked to or connected by one or more organic groups provides an excellent combination of properties for the purpose of a dental composition selected from a root canal sealing composition and a pulp capping composition when polymerized with a monomer having at least two groups selected from primary and secondary amino groups and thiol groups in a step-growth polymerization reaction. Specifically, favorable mechanical properties of the cured compositions, low viscosity, good handling properties and high dispersion stability of the uncured compositions as well as low dimensional changes of the compositions upon curing allow the replacement of bisphenol-A diglycidylether based materials and other epoxides in the preparation of a root canal sealing composition or pulp capping composition.

### Detained Description of Preferred Embodiments

The term "radioopacity" refers to a substance that will not allow X-rays or similar radiation to pass.

The term "organic group" relates to a moiety which may be based in general on a C₁₋₂₀ hydrocarbon group including aliphatic, alicyclic, or aromatic moieties or a combination thereof. The organic group links or connects further moieties. Accordingly, the organic group has a valency which corresponds to the number of further moieties.

Examples of aliphatic groups include alkyl groups. According to the invention, a C₁₋₂₀ alkyl group can include straight or branched alkyl groups having 1 to 20 carbon atoms, preferably 1 to 10 carbon atoms, for example, methyl, ethyl, n-propyl, isopropyl, n-butyl, isobutyl, sec-butyl, tert-butyl, n-pentyl, isopentyl and n-hexyl.

The alicyclic group may include, for example, a C₃₋₆ carbocyclic aliphatic ring, a C₃₋₆ heterocyclic aliphatic ring, a C₃₋₆ saturated aliphatic ring, or a C₃₋₆ unsaturated aliphatic ring. Examples of alicyclic groups include cycloalkyl or cycloalkylalkyl groups. A cycloalkyl group may be a C₃₋₆ cycloalkyl group. Examples of the cycloalkyl group can include those having 3 to 6 carbon atoms, for example, cyclopropyl, cyclobutyl, cyclopentyl and cyclohexyl. A cycloalkylalkyl group can include those having 4 to 8 carbon atoms. Examples for a cycloalkylalkyl group can include a combination of a straight or branched alkyl group having 1 to 6 carbon atoms and a cycloalkyl group having 3 to 6 carbon atoms. Examples of the cycloalkylalkyl group can for example, include methylcyclopropyl, methylcyclobutyl, methylcyclopentyl, methylcyclohexyl, ethylcyclopropyl, ethylcyclobutyl, ethylcyclopentyl, ethylcyclohexyl, propylcyclopropyl, propylcyclobutyl, and propylcyclopentyl.

An aromatic group may include a phenyl group or a naphtyl group.

Each of the C₁₋₂₀ hydrocarbon group may be a groups which may contain 1 to 6 heteroatoms selected from oxygen atoms and sulphur atoms. The heteroatoms may form part of a carbon chain of the C₁₋₂₀ hydrocarbon group, and/or the heteroatoms may form a functional group such as a carboxyl group, a hydroxyl group, a thiol group, a keto group, an ester group or a thioester group.

The present invention provides a dental composition. The dental composition may be generally any type of dental composition in which a bisphenol A based polymerizable component may be used. Specifically, the dental composition may be selected from a may be a root canal sealing composition or a pulp capping composition. Moreover, the dental compostion may also be a dental composite, a dental cement or a dental adhesive.

The dental composition comprises a specific polymerizable compound adapted to be polymerized in a step-growth polymerization reaction based on at least two optionally substituted 1, 3-dioxolane-2-one or 2,3-carbonatopropyl groups groups linked to or connected by one or more organic groups.

The polymerizable compound may obtained as a mixture or composition of stereo- and/or regioisomers depending on the nature of the precursor compounds. For the purpose of the present invention, a mixture or composition of polymerizable compounds may be used for preparing a dental composition. It is also possible to isolate a single stereo- and/or regioisomer of the polymerizable compound and to use the isolated single stereo- and/or regioisomer of the polymerizable compound for preparing a dental composition of the present invention.

The compound is polymerizable with the monomer (b) in a step-growth polymerization reaction based on at least two groups selected from the following formula (A) and (B) attached linked to or connected by one or more organic groups: 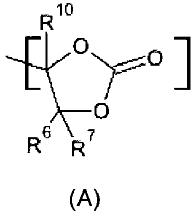 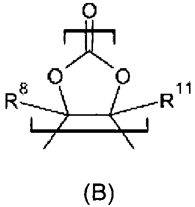 R⁶, R⁷ R⁸, R¹⁰ and R¹¹ which are independent from each other, represent a hydrogen atom or a C₁₋₆ alkyl group.

In the group (A), R⁶ and R⁷ are preferably selected from a hydrogen atom and a C₁₋₃ alkyl group such as a methyl, ethyl or propyl group. More preferably, R⁶ and R⁷ are selected from a hydrogen atom and a methyl or ethyl group. According to a preferred embodiment one of R⁶ and R⁷ is a hydrogen atom and the other is a C₁₋₆ alkyl group, preferably a C₁₋₃ alkyl group. According to a further preferred embodiment, R⁶ and R⁷ are a hydrogen atom.

In the group (A), R¹⁰ is preferably selected from a hygrogen atom and a C₁₋₃ alkyl group such as a methyl, ethyl or propyl group. More preferably, R¹⁰ is selected from a hydrogen atom and a methyl or ethyl group. According to a preferred embodiment R¹⁰ is a hydrogen atom or a methyl group.

In the group (B), R⁸ is preferably selected from a hygrogen atom and a C₁₋₃ alkyl group such as a methyl, ethyl or propyl group. More preferably, R⁸ is selected from a hydrogen atom and a methyl or ethyl group. According to a preferred embodiment R⁸ is a hydrogen atom or a methyl group.

In the group (B), R¹¹ is preferably selected from a hygrogen atom and a C₁₋₃ alkyl group such as a methyl, ethyl or propyl group. More preferably, R¹¹ is selected from a hydrogen atom and a methyl or ethyl group. According to a preferred embodiment R¹¹ is a hydrogen atom or a methyl group.

When the compound (c) having a group (A) is polymerized with the monomer (b), such as for example a compound having a primary amine functionality of the formula R^{x}NH₂ (wherein R^{x} is a residue providing in combination with the amino group a monomer (b)) in a step-growth polymerization reaction, the group (A) may be transformed into one of the following regioisomeric β-hydroxy urethane groups (A1) or (A2): 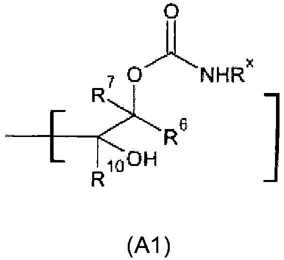 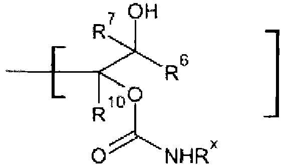 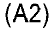 wherein R⁶, R⁷ and R¹⁰, which are independent from each other, represent a hydrogen atom or a C₁₋₆ alkyl group.

Moreover, when the compound (c) having a group (B) is polymerized with the monomer (b), such as for example a compound having a primary amine functionality of the formula R^{x}NH₂ (wherein R^{x} is a residue providing in combination with the amino group a monomer (b)) in a step-growth polymerization reaction, the group (B) may be transformed into one of the following regioisomeric β-hydroxy urethane groups (B1) or (B2): 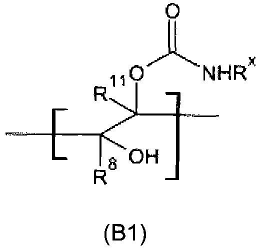 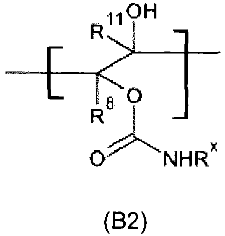 wherein R⁸ and R¹¹ which are independent from each other, represent a hydrogen atom or a C₁₋₆ alkyl group.

In case of a compound having one or more thiol groups, the corresponding regioisomeric β-hydroxy thioesters may be formed.

The compound (c) may be a compound obtainable by
(c1) reacting a mixture comprising
(i) (n+1) moles of one or more compounds of the following formula (I): 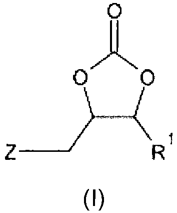 wherein
   Z is a hydroxyl group, a thiol group, or a group -NHR^{∘}, wherein R^{∘} is a C₁₋₆ alkyl group,
   R¹ is a hydrogen atom or a C₁₋₆ alkyl group,
(ii) per mole of a compound of the following formula (II): 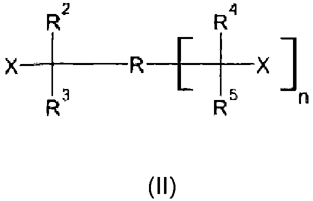 wherein
   R² and R³ which are independent from each other, represent a hydrogen atom or a C₁₋₆ alkyl group,
   the R⁴ and R⁵, which may be the same or different and which are independent from each other, represent a hydrogen atom or a C₁₋₆ alkyl group, or
   wherein two groups selected from R², R³ and the R⁴ and R⁵ together form a C₁₋₁₂ alkylene or C₁₋₁₂ alkyenlene group, respectively;
   the X, which may be the same or different, represent a group selected from a carboxylic acid halide group, an isocyanate group, and a leaving group,
   R is an (n+1)-valent organic group, and
   n is an integer of from 1 to 3,
   for forming a compound having 2,3-carbonatopropyl groups optionally substituted by R¹, which are polymerizable with the monomer (b) in a step-growth polymerization reaction.

In formula (I), Z is preferably a hydroxyl group, or a group -NHR°, wherein R^{∘} is a C₁₋₆ alkyl group.

In formula (I), R¹ is preferably a hydrogen atom or a C₁₋₃ alkyl group, more preferaby a hydrogen atom or a methyl group.

In formula (II), R² and R³ which are independent from each other, preferaly a hydrogen atom or a C₁₋₃ alkyl group, more preferaby a hydrogen atom or a methyl group.

In formula (II), the R⁴ and R⁵, which may be the same or different and which are independent from each other, preferably represent a hydrogen atom or a C₁₋₃ alkyl group, more preferaby a hydrogen atom or a methyl group.

In formula (II), two groups selected from R², R³ and the R⁴ and R⁵ together may form a C₁₋₁₂ alkylene or C₁₋₁₂alkyenlene group, respectively. Preferably, R², R³ and the R⁴ and R⁵ together may form a C₁₋₆ alkylene or C₁₋₆ alkyenlene group

In formula (II), the X, which may be the same or different, represent a group selected from a carboxylic acid halide group, an isocyanate group, and a leaving group. Preferably, the leaving group is selected from a halogen atom and a tosyl group.

In formula (II), R is an (n+1)-valent organic group, preferably a C₁₋₂₀ hydrocarbon group including aliphatic, alicyclic, or aromatic moieties or a combination thereof.

In case R is an aliphatic group, such group includes straight chain or branched alkylene groups. A C₁₋₂₀ alkylene group can include straight or branched alkyl groups having 1 to 20 carbon atoms, preferably 1 to 10 carbon atoms, for example, methylene, ethylene, n-propylene, isopropylene, n-butylene, isobutylene, sec-butylene, tert-butylene, n-pentylene, isopentylene and n-hexylene.

In case R is an alicyclic group, such group may include, for example, cycloalkylene or cycloalkylalkylene groups. A cycloalkylene group may be a C₃₋₆ cycloalkylne group. Examples of the cycloalkyl group can include those having 3 to 6 carbon atoms, for example, cyclopropylene, cyclobutylene, cyclopentylene and cyclohexylene. A cycloalkylalkylene group can include those having 4 to 8 carbon atoms. Examples for a cycloalkylalkylene group can include a combination of a straight or branched alkyl group having 1 to 6 carbon atoms and a cycloalkyl group having 3 to 6 carbon atoms. Examples of the cycloalkylalkylene group can for example, include methylcyclopropylene, methylcyclobutylene, methylcyclopentylene, methylcyclohexylene, ethylcyclopropylene, ethylcyclobutylene, ethylcyclopentylene, ethylcyclohexylene, propylcyclopropylene, propylcyclobutylene, and propylcyclopentylene.

An aromatic group may include a phenylene group or a naphtylene group.

Each of the (n+1)-valent organic groups for R may be a groups which may contain 1 to 6 heteroatoms selected from oxygen atoms and sulphur atoms. The heteroatoms preferably form part of a carbon chain of the C₁₋₂₀ hydrocarbon group, and/or the heteroatoms may form a functional group such as a carboxyl group, a hydroxyl group, a thiol group, a keto group, an ester group or a thioester group.

In formula (II), n is an integer of from 1 to 3, preferably 1 or 2.

Alternatively, the compound (c) may be a compound obtainable by
(c2) reacting by reacting carbon dioxide with an epoxy resin of the following formula (III): 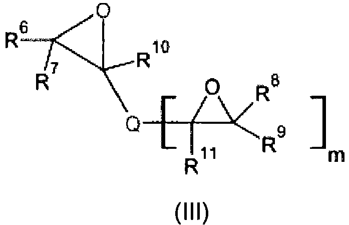 wherein
Q is an (m+1)-valent organic group,
R⁶ and R⁷ which are independent from each other, represent a hydrogen atom or a C₁₋₆ alkyl group,
the R⁸, and R⁹, which may be the same or different and which are independent from each other, represent a hydrogen atom or a C₁₋₆ alkyl group, or
wherein two groups selected from R⁶, R⁷, R¹⁰ and the R⁸, R⁹ and R¹¹ together form a C₁₋₁₂ alkylene or C₁₋₁₂ alkenylene group,
R¹⁰ and R¹¹ which are independent from each other, represent a hydrogen atom or a C₁₋₆ alkyl group, and
   m is an integer of from 1 to 3.

In formula (III), Q is an (m+1)-valent organic group, preferably a C₁₋₂₀ hydrocarbon group including aliphatic, alicyclic, or aromatic moieties or a combination thereof.

In case Q is an aliphatic group, such group includes alkylene groups. A C₁₋₂₀ alkylene group can include straight or branched alkyl groups having 1 to 20 carbon atoms, preferably 1 to 10 carbon atoms, for example, methylene, ethylene, n-propylene, isopropylene, n-butylene, isobutylene, sec-butylene, tert-butylene, n-pentylene, isopentylene and n-hexylene.

In case Q is an alicyclic group, such group may include, for example, cycloalkylene or cycloalkylalkylene groups. A cycloalkylene group may be a C₃₋₆ cycloalkylne group. Examples of the cycloalkyl group can include those having 3 to 6 carbon atoms, for example, cyclopropylene, cyclobutylene, cyclopentylene and cyclohexylene. A cycloalkylalkylene group can include those having 4 to 8 carbon atoms. Examples for a cycloalkylalkylene group can include a combination of a straight or branched alkyl group having 1 to 6 carbon atoms and a cycloalkyl group having 3 to 6 carbon atoms. Examples of the cycloalkylalkylene group can for example, include methylcyclopropylene, methylcyclobutylene, methylcyclopentylene, methylcyclohexylene, ethylcyclopropylene, ethylcyclobutylene, ethylcyclopentylene, ethylcyclohexylene, propylcyclopropylene, propylcyclobutylene, and propylcyclopentylene.

An aromatic group may include a phenylene group or a naphtylene group.

Each of the (n+1)-valent organic groups for Q may be a groups which may contain 1 to 6 heteroatoms selected from oxygen atoms and sulphur atoms. The heteroatoms preferably form part of a carbon chain of the C₁₋₂₀ hydrocarbon group, and/or the heteroatoms may form a functional group such as a carboxyl group, a hydroxyl group, a thiol group, a keto group, an ester group or a thiopester group.

In formula (III), R⁶ and R⁷ are as defined in case of formula (A) above. Preferably, R⁶ and R⁷ are selected from a hygrogen atom and a C₁₋₃ alkyl group such as a methyl, ethyl or propyl group. More preferably, R⁶ and R⁷ are selected from a hydrogen atom and a methyl or ethyl group. According to a preferred embodiment one of R⁶ and R⁷ is a hydrogen atom and the other is a C₁₋₆ alkyl group, preferably a C₁₋₃ alkyl group. According to a further preferred embodiment, R⁶ and R⁷ are a hydrogen atom.

In formula (III), R¹⁰ is as defined in case of formula (A) above. Preferably, R¹⁰ is selected from a hygrogen atom and a C₁₋₃ alkyl group such as a methyl, ethyl or propyl group. More preferably, R¹⁰ is selected from a hydrogen atom and a methyl or ethyl group. According to a preferred embodiment, R¹⁰ is selected from a hydrogen atom and a methyl group.

In formula (III), the R⁸ and R⁹, which may be the same or different and which are independent from each other, represent a hydrogen atom or a C₁₋₆ alkyl group R⁸ and R⁹ are selected from a hygrogen atom and a C₁₋₃ alkyl group such as a methyl, ethyl or propyl group. More preferably, R⁸ and R⁹ are selected from a hydrogen atom and a methyl or ethyl group. According to a preferred embodiment one of R⁸ and R⁹ is a hydrogen atom and the other is a C₁₋₆ alkyl group, preferably a C₁₋₃ alkyl group. According to a further preferred embodiment, R⁸ and R⁹ are a hydrogen atom.

In formula (III), R¹⁰ and R¹¹ which are independent from each other, represent a hydrogen atom or a C₁₋₆ alkyl group, more preferably a hygrogen atom or a C₁₋₃ alkyl group such as a methyl, ethyl or propyl group. More preferably, R¹⁰ and R¹¹ are selected from a hydrogen atom and a methyl or ethyl group.

In formula (III), two groups selected from R⁶, R⁷, R¹⁰ and the R⁸, R⁹ and R¹¹ together form a C₁₋₁₂ alkylene or C₁₋₁₂ alkenylene group, preferably C₁₋₆ alkylene or C₁₋₆ alkenylene group

In formula (III), m is an integer of from 1 to 3, preferably 1 or 2.

Alternatively, the compound (c) may be a compound obtainable by
(c3) reacting phosgene with the a polyol of the following formula (IV) 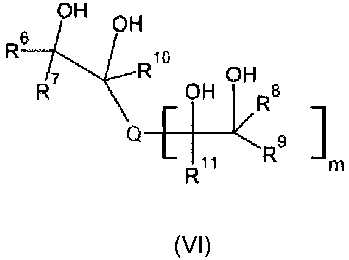 wherein Q, R⁶, R⁷, the R⁸, the R⁹, R¹⁰, the R¹¹, and m are independently as defined for formula (III).

According to a preferred embodiment, the compound step-growth polymerizable with the monomer (b) is a compound of the following formula (V): 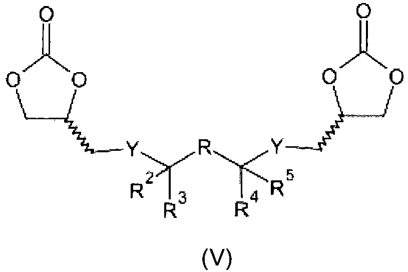 wherein R is as defined above and R², R³, R⁴ and R⁵ are independent from each other and represent a hydrogen atom or a methyl group and the Y, which are independent from each other represent a bond, an oxygen atom, a sulfur atom, an ester bond or a urethane bond, preferably wherein R is as defined in any one of claims 2 to 5.

In formula (V), R is preferably a straight chain or branched alkylene group or an aromatic froup. A C₁₋₂₀ alkylene group can include straight or branched alkyl groups having 1 to 20 carbon atoms, preferably 1 to 10 carbon atoms, for example, methylene, ethylene, n-propylene, isopropylene, n-butylene, isobutylene, sec-butylene, tert-butylene, n-pentylene, isopentylene and n-hexylene. The aromatic group is preferably a phenylene group or a xylylene group which may be substituted by one or more methyl groups.

The following compounds are particularly preferred:

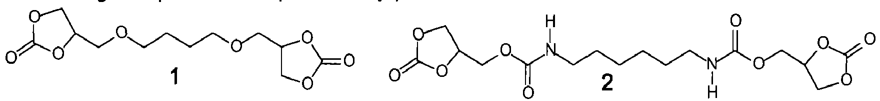

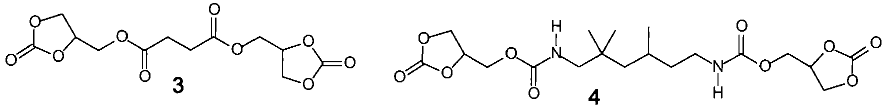

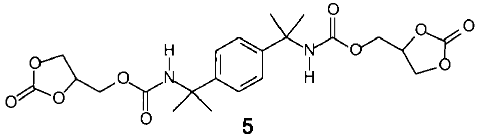

A dental composition selected from a root canal sealing composition and a pulp capping composition, contains beside the polymerizable compound of the present invention further components, namely a radioopaque particulate filler (a) and a monomer (b) having at least two groups selected from primary and secondary amino groups and thiol groups.

Suitable radioopaque particulate fillers may be selected from from fillers containing elements of the group comprising tungsten, bismuth, strontium, barium, tantalum, cerium, tin, zirconium, and yttrium.

The radioopaque particulate fillers may be surface modified by a surface modifying agent. The modifying compound is capable of reacting with surface atoms of the radioopaque particulate reactive glass, thereby forming a covalent bond between the surface atoms of the radioopaque particulate reactive glass and the modifying compound. The surface modifying agent may contain a modifying compound providing a dual function. For example, the modifying compound may contain one or more functional groups capable of taking part in a crosslinking reaction, thereby facilitating the additional crosslinking, whereby the cured cement has improved flexural strength and fracture toughness. The modifying agent may contain one or more modifying compounds.

Preferably, the surface modifying agent contains a hydrolyzable organofunctional silicon compound. Specific examples of modifying compounds contained in the surface modifying agent used in the present invention are 3-aminopropyltrimethoxysilane, 3-aminopropylmethyldimethoxysilane, 3-aminopropyldimethylmethoxysilane, 3-aminpropyltriethoxysilane, 3-aminopropylmethyldiethoxysilane, 3-aminopropyldimethylethoxysilane, 3-mercaptopropyltrimethoxysilane, 3-mercaptopropylmethyldimethoxysilane, 3-mercaptopropyldimethylmethoxysilane, 3-mercaptopropyltriethoxysilane, 3-mercaptopropylmethyldiethoxysilane, and 3-mercaptopropyldimethylethoxysilane. The compounds may be used alone or in combination of two or more different compounds.

The surface modifying agent may be used as such or dissolved or dispersed in a suitable solvent. Examples of suitable solvent are toluene, methanol, ethanol, isopropanol, and ethylacetate.

The radioopaque particulate filler usually has an average particle size of from 0.005 to 100 µm, preferably of from 0.01 to 40 µm as measured using, for example, by electron microscopy or by using a conventional laser diffraction particle sizing method as embodied by a MALVERN Mastersizer S or MALVERN Mastersizer 2000 apparatus. The radioopaque particulate reactive glass may be a multimodal radioopaque particulate reactive glass representing a mixture of two or more radioopaque particulate fractions having different average particle sizes. The radioopaque particulate reactive glass may also be a mixture of particles of different chemical composition. In particular, it is possible to use a mixture of a radioopaque particulate reactive material and a radioopaque particulate non-reactive material.

The dental composition selected from a root canal sealing composition and a pulp capping composition according to the invention preferably comprises 1 to 80 percent by weight, more preferably 40 to 70 percent by weight, of the radioopaque particulate filler, based on the weight of the entire composition.

Suitable monomers having at least two groups selected from primary and secondary amino groups and thiol groups may be selected from diamines or polyamines or polythiol compounds.

The diamines can be any diamine having primary and/or secondary amine groups. For some applications, primary diamines are especially useful. Representative diamines include aliphatic diamines such as ethylene diamine, 1,3-diaminopropane, 1,4-diaminobutane, 1,5-diaminopentane, 2-methyl-1,5-diaminopentane, 1,6-diaminohexane, bis(6-aminohexyl)ether, cycloaliphatic diamines such as isophorone diamine, 4,4'-methylene-bis-cyclohexylamine, bis(3-methyl-4-aminocyclohexyl)methane (BMACM), 2,2-bis(3-methyl-4-aminocyclohexyl)propane (BMACP), 2,6-bis(aminomethyl)norbornane (BAMN), and cyclohexane diamine, and heterocyclic diamines such as 3,4 diaminofuran and piperazine. Aromatic diamines such as N,N'-dibenzylethylenediamine, N,N'-dibenzyl-3,6-dioxaoctandiamine-1,8,N,N'-dibenzyl-5-oxanonandiamine-1,9,N,N'-dibenzyl-(2,2,4)/(2,4,4) trimethylhexamethylendiamine, m- or p-phenylenediamine, 2,4- or 2,6-diaminotoluene, and 4,4'-diaminodiphenylmethane may be used, but are less preferred for toxicological concerns. Therefore, it is preferred to utilize only non-aromatic amines. Mixtures of more than one diamine can also be utilized.

Polyamines which are useful as monomers are compounds having an average of more than two amine groups per molecule. Representative polyamines include diethylenetriamine, N,N'-dimethyldiethyltriamine, cyclohexyl-1,2,4-triamine, triethylenetetramine, cyclohexyl-1,2,4,5-tetramine, and tetramethylenepentamine. Polyamines can also be prepared by methods well known in the art such as by the polymerization of acrylic or other unsaturated monomers having primary or secondary amine functionality, or by the reaction of amines having at least two primary amine groups per molecule with a polycarboxylic acid to form polyamide amines. Other polyamines such as the Jeffamine ® polyoxypropyleneamines available from Huntsman Chemicals, Inc. are also practical. Mixtures of two or more additional polyamines can also be utilized.

Compounds having a thiol group which are useful as monomers, include aliphatic dithiols such as triethyleneglycol dithiol, or aromatic diamines such as o-, m- or p-xylylene dithiol.

A dental composition selected from a root canal sealing composition and a pulp capping composition may contain beside a polymerizable compound of the present invention further components which may be selected from polymerizable monomers, catalyst systems and conventional additives such as stabilizers and pigments.

Suitable catalyst systems amy contain an alkaline compound such as KOH, 1,4-diazabicyclo[2.2.2]octane (DABCO), or 4-dimethylaminopyridine.

The compound polymerizable with the monomer (b) in a step-growth polymerization reaction may be prepared by a reaction which comprises
(c1) reacting a mixture comprising
(i) at least (n+1) moles of one or more compounds of the following formula (I): 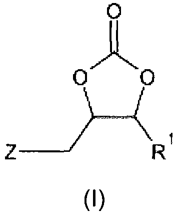 wherein
   Z is a hydroxyl group, a thiol group, or a group -NHR°, wherein R^{∘} is a C₁₋₆ alkyl group,
   R¹ is a hydrogen atom or a C₁₋₆ alkyl group, and
(ii) one mole of a compound of the following formula (II): 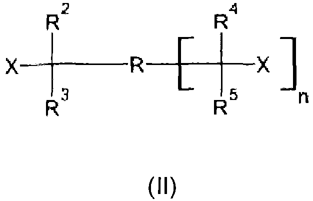 wherein
   R² and R³ which are independent from each other, represent a hydrogen atom or a C₁₋₆ alkyl group,
   the R⁴, and R⁵, which may be the same or different and which are independent from each other, represent a hydrogen atom or a C₁₋₆ alkyl group,
   the X, which may be the same or different, represent a group selected from a carboxylic acid halide group, an isocyanate group, and a leaving group,
   R is an (n+1)-valent organic group, and
   n is an integer of from 1 to 3.

As a specific example for the formation of urethane bonds, the following reaction may be mentioned:

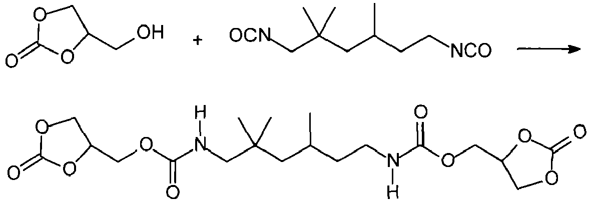

The reaction conditions for the reaction (c1) are not particularly limited. In general a compound of formula (II) may be preferably dissolved in a suitable solvent and a predetermined amount of a compound of formula (I) is added over a predetermined time at a predetermined temperature and optionally in the presence of a catalyst.

A suitable solvent for the compound of formula (II) may be an organic solvent and good solvent for the reactants. Specific examples are ethers such as diethylether, dioxane or tetrahydrofurane, aliphatic hydrocarbons such as pentane, hexane, heptane, octane, decane, isooctane, dodecane and hexadecane, and aromatic hydrocarbons such as benzene, toluene, and xylene a suitable mixture thereof.

The reaction temperature may be in the range of from -20°C to the boiling point of the solvent, preferably from -10 °C to room temperature (23°C). The reaction time may be adjusted in the range of from 30 minutes to 200 hours, preferably 1 to 150 hours.

A suitable catalyst for the formation of a urethane bond which enhances the isocyanate-hydroxyl reaction may be selected from dibutyltindilaurate, triethylene diamine, morpholine, N-ethylmorpholine, piperazine, triethanolamine, triethylamine, stannous octoate, dioctyl tin diacetate, lead octoate, stannous tallate and dibutyltindioxide. The amount of catalyst is in the range from 0.005 to 1 weight percent based on the total amount of diol or polyol and isocyanate. After the reaction, the product may be isolated by removing the solvent under reduced pressure and purified by using standard purification methods such as recrystallization, chromatography or distillation.

Alternatively, the compound polymerizable with the monomer (b) in a step-growth polymerization reaction may be prepared by a reaction which comprises (c2) reacting carbon dioxide with the an epoxy resin of the following formula (III): 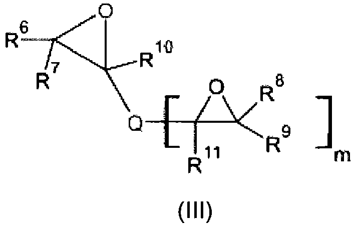 wherein
Q is an (m+1)-valent organic group,
R⁶ and R⁷ which are independent from each other, represent a hydrogen atom or a C₁₋₆ alkyl group,
the R⁸, and R⁹, which may be the same or different and which are independent from each other, represent a hydrogen atom or a C₁₋₆ alkyl group,
m is an integer of from 1 to 3.
The reaction conditions for the reaction (c2) are not particularly limited. In general a compound of formula (III) may be preferably dissolved in a suitable solvent and a predetermined amount of carbon dioxide is added over a predetermined time at a predetermined temperature and optionally in the presence of a catalyst.

The reaction temperature may be in the range of from -20°C to the boiling point of the solvent, preferably from -10 °C to room temperature (23°C). The reaction time may be adjusted in the range of from 30 minutes to 200 hours, preferably 1 to 150 hours.

A suitable catalyst for the formation of a cyclic carbonate which enhances the epoxide-carbon dioxide reaction may be selected from zinc halides. The amount of catalyst is in the range from 0.005 to 10 weight percent based on the total amount of carbon dioxide and compound of formula (III). After the reaction, the product may be isolated by removing the solvent under reduced pressure and purified by using standard purification methods such as recrystallization, chromatography or distillation.

Alternatively, the compound polymerizable with the monomer (b) in a step-growth polymerization reaction may be prepared by a reaction which comprises (c3) reacting phosgene with the a polyol of the following formula (IV) 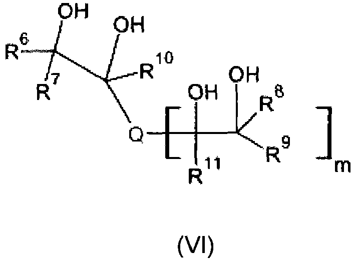 wherein Q, R⁶, R⁷, the R⁸, the R⁹, R¹⁰, the R¹¹, and m are independently as defined for formula (III).

The reaction conditions for the reaction (c3) are not particularly limited. In general a compound of formula (IV) may be preferably dissolved in a suitable solvent and a predetermined amount of phosgene or a derivative thereof is added over a predetermined time at a predetermined temperature and optionally in the presence of a catalyst.

The reaction temperature may be in the range of from -20°C to the boiling point of the solvent, preferably from -10 °C to room temperature (23°C). The reaction time may be adjusted in the range of from 30 minutes to 200 hours, preferably 1 to 150 hours.

A suitable catalyst for the formation of a cyclic carbonate which enhances the diol-phosgene reaction may be selected from zinc halides. The amount of catalyst is in the range from 0.005 to 10 weight percent based on the total amount of carbon dioxide and compound of formula (III). After the reaction, the product may be isolated by removing the solvent under reduced pressure and purified by using standard purification methods such as recrystallization, chromatography or distillation.

The compound polymerizable with the monomer (b) in a step-growth polymerization reaction may be used for the preparation of a dental composition selected from a root canal sealing composition, and a pulp capping composition.

### Examples

The present invention will be further illustrated with the following examples.

### Example 1 (Structure 4)

### Bis-(2,3-carbonatopropyl)-2,2,4-(2,4,4)-trimethyl-hexamethylendicarbamate

In a 3 neck round bottom flask 50 ml of THF were cooled in an ice bath. To the solvent 5.1 g (24.25 mmol) trimethyl-hexamethylen diisocyanate, 25 mg (0.04 mmol) dibutyltin dilaurate and 5.7 g (48.5 mmol) glycerolcarbonate were added. The reaction was kept under ice cooling for further 5 hours. After removing the ice bath the reactions media was stirred at RT until no isocyanate signals could be observed by IR Spectroscopy (96h).

After reducing the solvent under reduced pressure 100 ml of petrol ether (60/80) were added and the mixture was further stirred for 10 minutes. From the precipitating high viscous gel the solvent was removed and the product was further dried under reduced pressure at 30°C.
Yield: 10.8 g
|n*₀|[Pa^{∗}s]: 307 (25°C)
MS [ESI]: 447 [M+H⁺]
IR [cm⁻¹]: 2957, 2886 (Alkyl-Chain), 1792 (Carbonate), 1714 (Urethane)
¹³C NMR [75 MHz, CDCl₃]: δ 156.07 (**Pos. 19**), 156.07 (**Pos. 4**), 154.07 (**Pos. 12,26**), 74,65 (**Pos.1,23**), 66,09 (**Pos. 10,24**), 63,43 (**Pos.2,22**), 48.80- 46.25- 45.66- 45.41- 41.12- 39.11-37.45- 35.17- 32.92-29.50-27.99- 26.26- 25.69- 25.23- 22.37- 20.80 **(Alkyl-chain of the 2,2,4-(4,4,2)- Isomers)**

### Example 2

### Bis-(2,3-carbonatopropyl)-hexamethylendicarbamate

In a 3 neck round bottom flask 50 ml of THF were cooled in an ice bath. To the solvent 4.1 g (24.25 mmol) hexamethylen diisocyanate, 25 mg (0.04 mmol) dibutyltin dilaurate and 5.7 g (48.5 mmol) glycerolcarbonate were added. The reaction was kept under ice cooling for further 5 hours. After removing the ice bath the reactions media was stirred at RT until no isocyanate signals could be observed by IR Spectroscopy (96h).

From the obtained reactions media the solvent was removed under reduced pressure. The resulting white solid was isolated and washed throughout by petrol ether (60/80). The obtained product was further dried under reduced pressure at 30°C.
Yield: 9.8 g
MS [EI]: 404 [M⁺]
IR [cm⁻¹]: 2938, 2869 (Alkyl-Chain), 1783 (Carbonate), 1688 (Urethane)
¹³C NMR [75 MHz, DMSO]: δ 155.63 (**Pos. 4,19**), 154.73 (**Pos. 12,26**), 74.89 (**Pos. 1,23**), 65.90 (**Pos. 10,24**), 63.07(**Pos. 2,22**), 40,27 (**Pos. 6,17**), 29.25 (**Pos.7,16**), 25.91(**Pos. 8,9**).

### Example 3

### Bis-(2,3-carbonatopropyl)-adipic acid diester

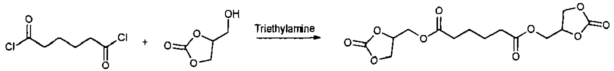

To cooled mixture of 11.6 ml (137.4 mmol) glycerol carbonate and 19 ml (137.4 mmol) triethylamine in 80 ml chloroform were added drop by drop 10 ml (68.7 mmol) adipic acid dichloride and stirred for 4 h. Thereafter the reaction mixture was stirred for addition 20 h at room temperature. The solution was extracted with 2.5 L water and filtered over Al₂O₂. After evaporation of the solvent a beige solid was received.

### Application Example 1

The geltime at 37 °C of a stoichiometric mixture of bis- (2,3-Carbonatopropyl)- 2,2,4 bzw. 2,4,4 Trimethyl-hexamethylendicarbamate (Example 1) and hexamethylene diamine is 20-30 min.

### Comparison Example 1

The geltime at 37 °C of a stoichiometric mixture of 1,4-butandiol diglycidyl ether and hexamethylene diamine is 20 h.

### Comparison Example 2

The geltime at 37 °C of a stoichiometric mixture of 2,2-Bis[4-(2,3-epoxypropoxy)phenyl] propane and hexamethylene diamine is 3.6 h.

## Claims

1. Dental composition selected from a root canal sealing composition and a pulp capping composition, comprising:
(a) a radioopaque particulate filler;
(b) a monomer having at least two groups selected from primary and secondary amino groups and thiol groups; and
(c) a compound polymerizable with the monomer (b) in a step-growth polymerization reaction based on at least two groups selected from the following formula (A) and (B) attached to or connected by one or more organic groups: 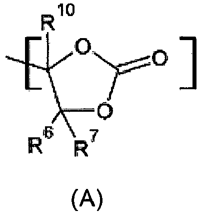 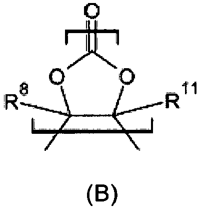 wherein R⁶, R⁷ R⁸, R¹⁰ and R¹¹, which are independent from each other, represent a hydrogen atom or a C₁₋₆ alkyl group.

2. The dental composition according to claim 1, wherein the compound polymerizable with the monomer (b) in a step-growth polymerization reaction is a compound obtainable by
(c1) reacting a mixture comprising
(i) (n+1) moles of one or more compounds of the following formula (I): 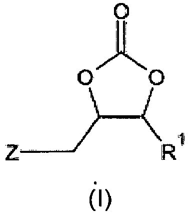 wherein
Z is a hydroxyl group, a thiol group, or a group -NHR°, wherein R^{∘} is a C₁₋₆ alkyl group,
R¹ is a hydrogen atom or a C₁₋₆ alkyl group,
(ii) per mole of a compound of the following formula (II): 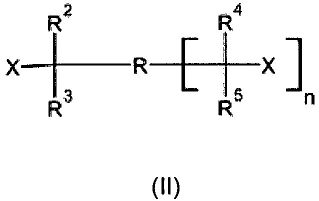 wherein
R² and R³ which are independent from each other, represent a hydrogen atom or a C₁₋₆ alkyl group,
the R⁴ and R⁵, which may be the same or different and which are independent from each other, represent a hydrogen atom or a C₁₋₆ alkyl group, or
wherein two groups selected from R², R³ and the R⁴ and R⁵ together form a C₁₋₁₂ alkylene or C₁₋₁₂ alkyenlene group, respectively; the X, which may be the same or different, represent a group selected from a carboxylic acid halide group, an isocyanate group, and a leaving group,
R is an (n+1)-valent organic group, and
n is an integer of from 1 to 3,
for forming a compound having 2,3-carbonatopropyl groups optionally substituted by R¹, which are polymerizable with the monomer (b) in a step-growth polymerization reaction; and/or
(c2) reacting carbon dioxide with an epoxy resin of the following formula (III): 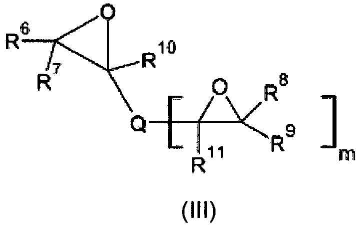 wherein
Q is an (m+1)-valent organic group,
R⁶ and R⁷ which are independent from each other, represent a hydrogen atom or a C₁₋₆ alkyl group,
the R⁸, and R⁹, which may be the same or different and which are independent from each other, represent a hydrogen atom or a C₁₋₆ alkyl group, or
wherein two groups selected from R⁶, R⁷, R¹⁰ and the R⁸, R⁹ and R¹¹ together form a C₁₋₁₂ alkylene or C₁₋₁₂ alkyenlene group,
R¹⁰ and R¹¹ which are independent from each other, represent a hydrogen atom or a C₁₋₆ alkyl group, and
m is an integer of from 1 to 3; and/or
(c3) reacting phosgene with the a polyol of the following formula (IV) 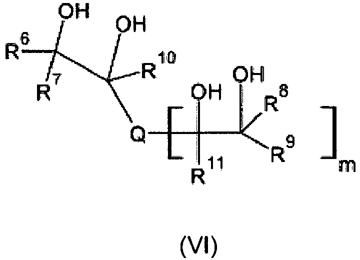 wherein Q, R⁶, R⁷, the R⁸, the R⁹, R¹⁰, the R¹¹, and m are independently as defined for formula (III).

3. The dental composition according to claim 2, wherein R is an (n+1)-valent hydrocarbon group having 2 to 20 carbon atoms which may contain from 1 to 6 heteroatoms and/or groups selected from oxygen atoms, sulfur atoms, tertiary amino groups or amide groups, whereby the heteroatoms and may form part of a carbon chain or a functional group selected from hydroxyl groups, thiol groups, carbonyl groups, or thiocarbonyl groups.

4. The dental composition according to claim 2, wherein Q is an (m+1)-valent hydrocarbon group having 2 to 20 carbon atoms which may contain from 1 to 6 heteroatoms and/or groups selected from oxygen atoms, sulfur atoms, tertiary amino groups or amide groups, whereby the heteroatoms and may form part of a carbon chain or a functional group selected from hydroxyl groups, thiol groups, carbonyl groups, or thiocarbonyl groups.

5. The dental composition according to claim 3 or 4, wherein R is an (n+1)-valent aliphatic, alicyclic or aromatic hydrocarbon hydrocarbon group which may contain from 1 to 6 heteroatoms selected from oxygen atoms and sulfur atoms, or Q is an (m+1)-valent aliphatic, alicyclic or aromatic hydrocarbon hydrocarbon group which may contain from 1 to 6 heteroatoms and/or groups selected from oxygen atoms, sulfur atoms, tertiary amino groups or amide groups, whereby the heteroatoms and may form part of a carbon chain or a functional group selected from hydroxyl groups, thiol groups, carbonyl groups, or thiocarbonyl groups.

6. The dental composition according to claim 2, wherein R or Q is a straight-chain or branched aliphatic hydrocarbon group having 2 to 20 carbon atoms and which which may contain from 1 to 6 heteroatoms and/or groups selected from oxygen atoms, sulfur atoms, tertiary amino groups or amide groups, whereby the heteroatoms and may form part of a carbon chain or a functional group selected from hydroxyl groups, thiol groups, carbonyl groups, or thiocarbonyl groups.

7. The dental composition according to any one of the preceding claims, wherein R or Q is an aromatic group.

8. The dental composition according to any one of the preceding claims, wherein R¹ or each of R⁶ to R⁹ is a hydrogen atom.

9. The dental composition according to any one of the preceding claims, wherein R² and R³ which are independent from each other, represent a hydrogen atom or a methyl group.

10. The dental composition according to any one of the preceding claims, wherein the R⁴ and R⁵, which may be the same or different and which are independent from each other, represent a hydrogen atom or a methyl group, or
wherein n or m are 1.

11. The dental composition according to any one of the preceding claims, wherein the compound step-growth polymerizable with the monomer having at least two groups selected from primary and secondary amino groups and thiol groups is a compound of the following formula (V): 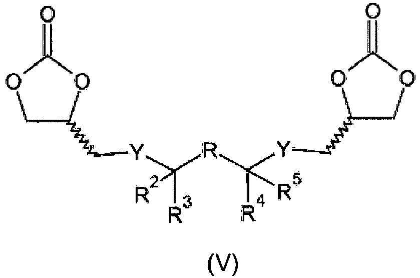 wherein R is as defined in claim 1 and R², R³, R⁴ and R⁵ are independent from each other and represent a hydrogen atom or a methyl group and the Y, which are independent from each other represent a bond, an oxygen atom, a sulfur atom, an ester bond or a urethane bond, preferably wherein R is as defined in any one of claims 2 to 5.

12. A process for the preparation of a compound polymerizable with a monomer having at least two groups selected from primary and secondary amino groups and thiol groups in a step-growth polymerization reaction based on at least two groups selected from the following formula (A) and (B) attached to or connected by one or more organic groups: 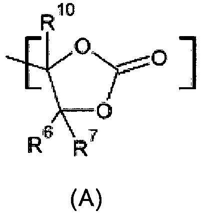 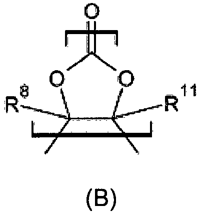 R⁶, R⁷ R⁸, R¹⁰ and R¹¹ which are independent from each other, represent a hydrogen atom or a C₁₋₆ alkyl group
which process comprises
(c1) reacting a mixture comprising
(i) at least (n+1) moles of one or more compounds of the following formula (I): 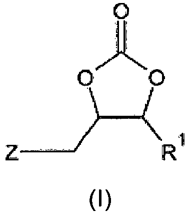 wherein
Z is a hydroxyl group, a thiol group, or a group -NHR°, wherein R° is a C₁₋₆ alkyl group,
R¹ is a hydrogen atom or a C₁₋₆ alkyl group, and
(ii) one mole of a compound of the following formula (II): 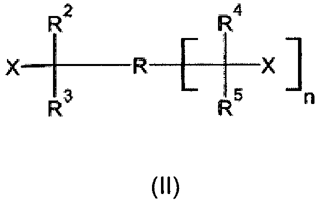 wherein
R² and R³ which are independent from each other, represent a hydrogen atom or a C₁₋₆ alkyl group,
the R⁴, and R⁵, which may be the same or different and which are independent from each other, represent a hydrogen atom or a C₁₋₆ alkyl group,
the X, which may be the same or different, represent a group selected from a carboxylic acid halide group, an isocyanate group, and a leaving group,
R is an (n+1)-valent organic group, and
n is an integer of from 1 to 3;
(c2) reacting carbon dioxide with the an epoxy resin of the following formula (III): 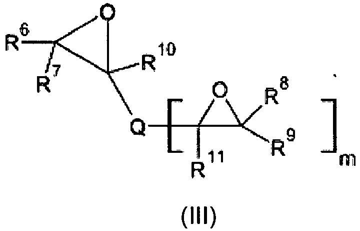 wherein
Q is an (m+1)-valent organic group,
R⁶ and R⁷ which are independent from each other, represent a hydrogen atom or a C₁₋₆ alkyl group,
the R⁸, and R⁹, which may be the same or different and which are independent from each other, represent a hydrogen atom or a C₁₋₆ alkyl group,
m is an integer of from 1 to 3; and/or
(c3) reacting phosgene with the a polyol of the following formula (IV) 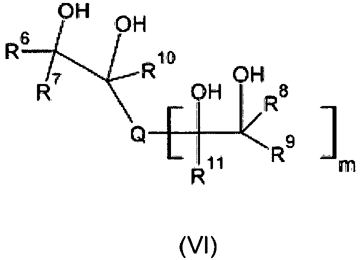 wherein Q, R⁶, R⁷, the R⁸, the R⁹, R¹⁰, the R¹¹, and m are independently as defined for formula (III).

13. Use of the compound polymerizable with a monomer having at least two groups selected from primary and secondary amino groups and thiol groups in a step-growth polymerization reaction based on at least two groups selected from the following formula (A) and (B) attached to or connected by one or more organic groups: 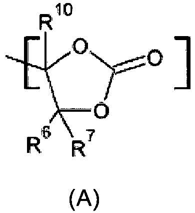 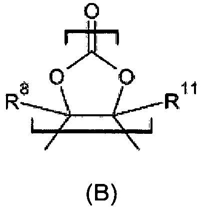 R⁶, R⁷ R⁸, R¹⁰ and R¹¹ which are independent from each other, represent a hydrogen atom or a C₁₋₆ alkyl group
which compound is obtainable by
(c1) reacting a mixture comprising
(i) at least (n+1) moles of one or more compounds of the following formula (I): 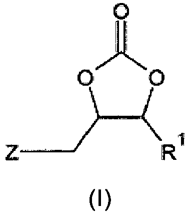 wherein
Z is a hydroxyl group, a thiol group, or a group -NHR°, wherein R^{∘} is a C₁₋₆ alkyl group,
R¹ is a hydrogen atom or a C₁₋₆ alkyl group,
(ii) one mole of a compound of the following formula (II): 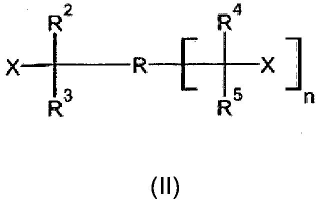 wherein
R² and R³ which are independent from each other, represent a hydrogen atom or a C₁₋₆ alkyl group,
the R⁴, and R⁵, which may be the same or different and which are independent from each other, represent a hydrogen atom or a C₁₋₆ alkyl group,
the X, which may be the same or different, represent a group selected from a carboxylic acid halide group, an isocyanate group, and a leaving group,
R is an (n+1)-valent organic group, and
n is an integer of from 1 to 3; or
(c2) by reacting carbon dioxide with the an epoxy resin of the following formula (III): 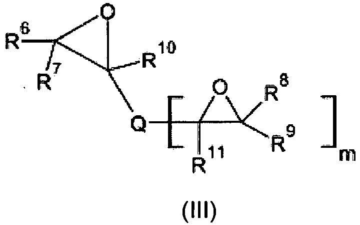 wherein
Q is an (m+1)-valent organic group,
R⁶ and R⁷ which are independent from each other, represent a hydrogen atom or a C₁₋₆ alkyl group,
the R⁸, and R⁹, which may be the same or different and which are independent from each other, represent a hydrogen atom or a C₁₋₆ alkyl group,
m is an integer of from 1 to 3; and/or
(c3) reacting phosgene with the a polyol of the following formula (IV) 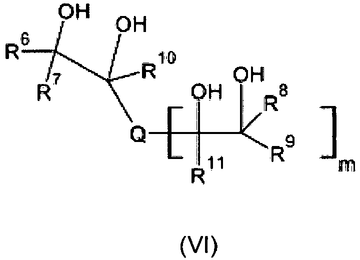 wherein Q, R⁶, R⁷, the R⁸, the R⁹, R¹⁰, the R¹¹, and m are independently as defined for formula (III), for the preparation of a dental composition selected from a root canal sealing composition and a pulp capping composition.

## Patentansprüche

1. Dentalzusammensetzung, die aus einer Wurzelkanalfüllungszusammensetzung und einer Zusammensetzung zur Pulpaüberkappung ausgewählt ist, umfassend:
(a) einen röntgenstrahlenundurchlässigen, teilchenförmigen Füllstoff;
(b) ein Monomer, das mindestens zwei Gruppen aufweist, die aus primären und sekundären Aminogruppen und Thiolgruppen ausgewählt sind; und
(c) eine Verbindung, die mit dem Monomer (b) in einer Stufenwachstumspolymerisationsreaktion polymerisiert werden kann, die auf mindestens zwei Gruppen basiert, die aus der folgenden Formel (A) und (B) ausgewählt sind, die durch ein oder mehrere organische Gruppen verbunden oder daran gebunden sind: 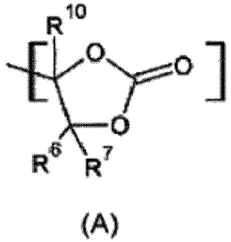 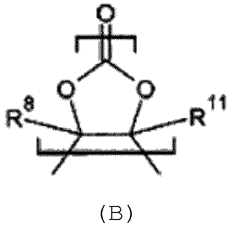 wobei R⁶, R⁷ R⁸, R¹⁰ und R¹¹, die voneinander unabhängig sind, ein Wasserstoffatom oder eine C₁₋₆ Alkylgruppe darstellen.

2. Dentalzusammensetzung nach Anspruch 1, wobei die Verbindung, die mit dem Monomer (b) in einer Stufenwachstumspolymerisationsreaktion polymerisiert werden kann, eine Verbindung ist, die erhalten werden kann durch
(c1) Reagieren einer Mischung, die Folgendes umfasst
(i) (n+1) Mol einer oder mehreren Verbindungen der folgenden Formel (I): 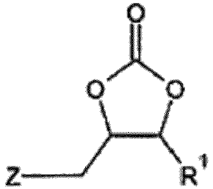 wobei
Z eine Hydroxylgruppe, eine Thiolgruppe oder eine Gruppe -NHR° ist, wobei R° eine C₁₋₆ Alkylgruppe ist,
R¹ ein Wasserstoffatom oder eine C₁₋₆ Alkylgruppe ist,
(ii) pro Mol einer Verbindung der folgenden Formel (II) : 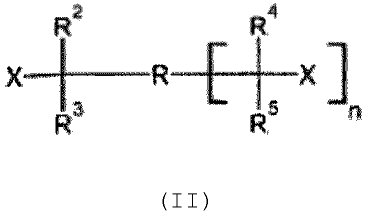 wobei
R² und R³, die voneinander unabhängig sind, ein Wasserstoffatom oder eine C₁₋₆ Alkylgruppe darstellen,
R⁴ und R⁵, die gleich oder verschieden sein können und die voneinander unabhängig sind, ein Wasserstoffatom oder eine C₁₋₆ Alkylgruppe darstellen, oder
wobei zwei Gruppen, die aus R², R³ und R⁴ und R⁵ ausgewählt sind, zusammen ein C₁₋₁₂ Alkylen bzw. eine C₁₋₁₂ Alkylengruppe bilden;
X, das gleich oder verschieden sein kann, eine Gruppe darstellt, die aus einer Carbonsäurehalogenidgruppe, einer Isocyanatgruppe und einer Abgangsgruppe ausgewählt ist,
R eine (n+1)-valente organische Gruppe ist, und
n eine ganze Zahl von 1 bis 3 ist,
zum Bilden einer Verbindung, die 2,3-Carbonatpropylgruppen aufweist, optional substituiert durch R¹, die mit dem Monomer (b) in einer Stufenwachstumspolymerisationsreaktion polymerisiert werden können; und/oder
(c2) Reagieren von Kohlendioxid mit einem Epoxidharz der folgenden Formel (III): 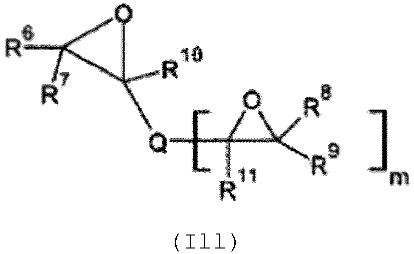 wobei
Q eine (m+1)-valente organische Gruppe ist,
R⁶ und R⁷, die voneinander unabhängig sind, ein Wasserstoffatom oder eine C₁₋₆ Alkylgruppe darstellen,
R⁸ und R⁹, die gleich oder verschieden sein können und die voneinander unabhängig sind, ein Wasserstoffatom oder eine C₁₋₆ Alkylgruppe darstellen, oder
wobei zwei Gruppen, die aus R⁶, R⁷, R¹⁰ und R⁸, R⁹ und R¹¹ ausgewählt sind, zusammen ein C₁₋₁₂ Alkylen oder eine C₁₋₁₂ Alkylengruppe bilden,
R¹⁰ und R¹¹, die voneinander unabhängig sind, ein Wasserstoffatom oder eine C₁₋₆ Alkylgruppe darstellen, und
m eine ganze Zahl von 1 bis 3 ist; und/oder
(c3) Reagieren von Phosgen mit einem Polyol der folgenden Formel (IV) 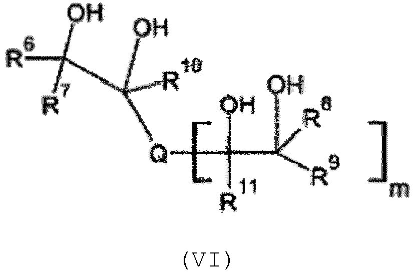 wobei Q, R⁶, R⁷, R⁸, R⁹, R¹⁰, R¹¹ und m unabhängig sind, wie definiert für Formel (III).

3. Dentalzusammensetzung nach Anspruch 2, wobei R eine (n+1)-valente Kohlenwasserstoffgruppe ist, die 2 bis 20 Kohlenstoffatome aufweist, die 1 bis 6 Heteroatome und/oder Gruppen enthalten kann, die aus Sauerstoffatomen, Schwefelatomen, tertiären Aminogruppen oder Amidgruppen ausgewählt sind, wobei die Heteroatome Teil einer Kohlenstoffkette oder einer funktionellen Gruppe sein können, die aus Hydroxylgruppen, Thiolgruppen, Carbonylgruppen oder Thiocarbonylgruppen ausgewählt ist.

4. Dentalzusammensetzung nach Anspruch 2, wobei Q eine (m+1)-valente Kohlenwasserstoffgruppe ist, die 2 bis 20 Kohlenstoffatome aufweist, die von 1 bis 6 Heteroatome und/oder Gruppen enthalten kann, die aus Sauerstoffatomen, Schwefelatomen, tertiären Aminogruppen oder Amidgruppen ausgewählt sind, wobei die Heteroatome Teil einer Kohlenstoffkette oder einer funktionellen Gruppe sein können, die aus Hydroxylgruppen, Thiolgruppen, Carbonylgruppen oder Thiocarbonylgruppen ausgewählt ist.

5. Dentalzusammensetzung nach Anspruch 3 oder 4, wobei R eine (n+1)-valente aliphatische, alizyklische oder aromatische Kohlenwasserstoffgruppe ist, die 1 bis 6 Heteroatome enthalten kann, die aus Sauerstoffatomen und Schwefelatomen ausgewählt sind, oder Q eine (m+1)-valente aliphatische, alizyklische oder aromatische Kohlenwasserstoff-Kohlenwasserstoffgruppe ist, die 1 bis 6 Heteroatome und/oder Gruppen enthalten kann, die aus Sauerstoffatomen, Schwefelatomen, tertiären Aminogruppen oder Amidgruppen ausgewählt sind, wobei die Heteroatome Teil einer Kohlenstoffkette oder einer funktionellen Gruppe sein können, die aus Hydroxylgruppen, Thiolgruppen, Carbonylgruppen oder Thiocarbonylgruppen ausgewählt ist.

6. Dentalzusammensetzung nach Anspruch 2, wobei R oder Q eine geradkettige oder verzweigte aliphatische Kohlenwasserstoffgruppe ist, die 2 bis 20 Kohlenstoffatome aufweist und die 1 bis 6 Heteroatome und/oder und Gruppen enthalten kann, die aus Sauerstoffatomen, Schwefelatomen, tertiären Aminogruppen oder Amidgruppen ausgewählt sind, wobei die Heteroatome Teil einer Kohlenstoffkette oder einer funktionellen Gruppe sein können, die aus Hydroxylgruppen, Thiolgruppen, Carbonylgruppen oder Thiocarbonylgruppen ausgewählt ist.

7. Dentalzusammensetzung nach einem der vorstehenden Ansprüche, wobei R oder Q eine aromatische Gruppe ist.

8. Dentalzusammensetzung nach einem der vorstehenden Ansprüche, wobei R¹ oder jedes von R⁶ bis R⁹ ein Wasserstoffatom ist.

9. Dentalzusammensetzung nach einem der vorstehenden Ansprüche, wobei R² und R³, die voneinander unabhängig sind, ein Wasserstoffatom oder eine Methylgruppe darstellen.

10. Dentalzusammensetzung nach einem der vorstehenden Ansprüche, wobei R⁴ und R⁵, die gleich oder verschieden sein können und die voneinander unabhängig sind, ein Wasserstoffatom oder eine Methylgruppe darstellen, oder wobei n oder m 1 sind.

11. Dentalzusammensetzung nach einem der vorstehenden Ansprüche, wobei die Verbindung, die durch Stufenwachstum mit dem Monomer polymerisiert werden kann, das zwei Gruppen aufweist, die aus primären und sekundären Aminogruppen und Thiolgruppen ausgewählt sind, eine Verbindung der folgenden Formel (V) ist: 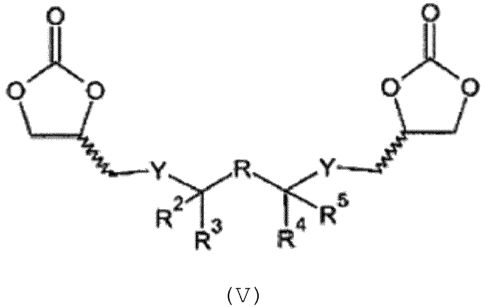 wobei R wie in Anspruch 1 definiert ist, und R², R³, R⁴ und R⁵ jeweils unabhängig voneinander sind und ein Wasserstoffatom oder eine Methylgruppe darstellen und die Y, die jeweils unabhängig voneinander eine Bindung, ein Sauerstoffatom, ein Schwefelatom, eine Esterbindung oder eine Urethanbindung darstellen, bevorzugt wobei R wie in einem der Ansprüche 2 bis 5 definiert ist.

12. Prozess zur Herstellung einer Verbindung, die mit einem Monomer, das mindestens zwei Gruppen aufweist, die aus primären und sekundären Aminogruppen und Thiolgruppen ausgewählt sind, in einer Stufenwachstumspolymerisationsreaktion polymerisiert werden kann, die auf mindestens zwei Gruppen basiert, die aus der folgenden Formel (A) und (B) ausgewählt sind, die durch ein oder mehrere organische Gruppen verbunden oder daran gebunden sind: 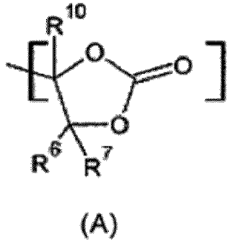 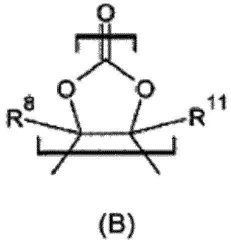 R⁶, R⁷ R⁸, R¹⁰ und R¹¹, die unabhängig voneinander sind, ein Wasserstoffatom oder eine C₁₋₆ Alkylgruppe darstellen,
wobei der Prozess Folgendes umfasst
(c1) Reagieren einer Mischung, die Folgendes umfasst
(i) mindestens (n+1) Mol einer oder mehreren Verbindungen der folgenden Formel (I): 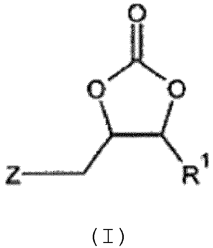 wobei
Z eine Hydroxylgruppe, eine Thiolgruppe oder eine Gruppe -NHR° ist, wobei R° eine C₁₋₆ Alkylgruppe ist,
R¹ ein Wasserstoffatom oder eine C₁₋₆ Alkylgruppe ist, und
(ii) ein Mol einer Verbindung der folgenden Formel (II) : 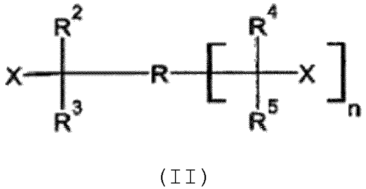 wobei
R² und R³, die voneinander unabhängig sind, ein Wasserstoffatom oder eine C₁₋₆ Alkylgruppe darstellen,
R⁴ und R⁵, die gleich oder verschieden sein können und die voneinander unabhängig sind, ein Wasserstoffatom oder eine C₁₋₆ Alkylgruppe darstellen,
X, das gleich oder verschieden sein kann, eine Gruppe darstellt, die aus einer Carbonsäurehalogenidgruppe, einer Isocyanatgruppe und einer Abgangsgruppe ausgewählt ist,
R eine (n+1)-valente organische Gruppe ist, und
n eine ganze Zahl von 1 bis 3 ist;
(c2) Reagieren von Kohlendioxid mit einem Epoxidharz der folgenden Formel (III): 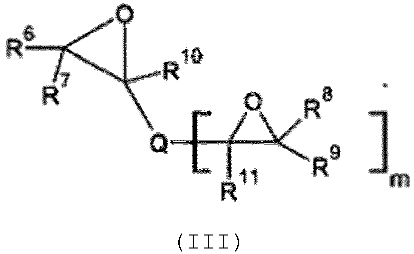 wobei
Q eine (m+1)-valente organische Gruppe ist,
R⁶ und R⁷, die voneinander unabhängig sind, ein Wasserstoffatom oder eine C₁₋₆ Alkylgruppe darstellen,
R⁸ und R⁹, die gleich oder verschieden sein können und die voneinander unabhängig sind, ein Wasserstoffatom oder eine C₁₋₆ Alkylgruppe darstellen,
m eine ganze Zahl von 1 bis 3 ist; und/oder
(c3) Reagieren von Phosgen mit einem Polyol der folgenden Formel (IV) 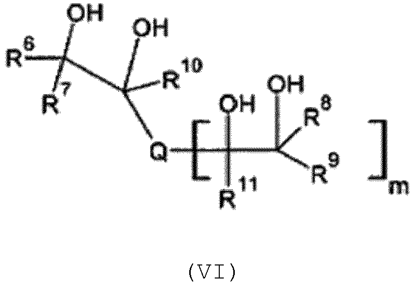 wobei Q, R⁶, R⁷, R⁸, R⁹, R¹⁰, R¹¹ und m unabhängig sind, wie definiert für Formel (III).

13. Verwendung der Verbindung, die mit einem Monomer, das mindestens zwei Gruppen aufweist, die aus primären und sekundären Aminogruppen und Thiolgruppen ausgewählt sind, in einer Stufenwachstumspolymerisationsreaktion polymerisiert werden kann, die auf mindestens zwei Gruppen basiert, die aus der folgenden Formel (A) und (B) ausgewählt sind, die durch ein oder mehrere organische Gruppen verbunden oder daran gebunden sind: 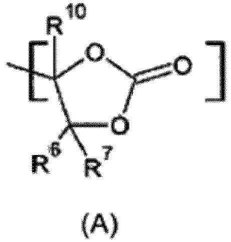 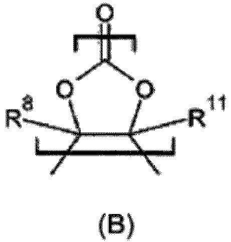 R⁶, R⁷ R⁸, R¹⁰ und R¹¹, die unabhängig voneinander sind, ein Wasserstoffatom oder eine C₁₋₆ Alkylgruppe darstellen,
wobei die Verbindung erhalten werden kann durch
(c1) Reagieren einer Mischung, die Folgendes umfasst
(i) mindestens (n+1) Mol einer oder mehreren Verbindungen der folgenden Formel (I): 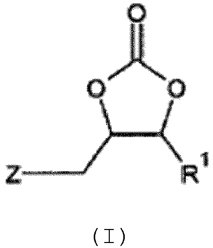 wobei
Z eine Hydroxylgruppe, eine Thiolgruppe oder eine -NHR° Gruppe ist, wobei R° eine C₁₋₆ Alkylgruppe ist,
R¹ ein Wasserstoffatom oder eine C₁₋₆ Alkylgruppe ist,
(ii) ein Mol einer Verbindung der folgenden Formel (II) : 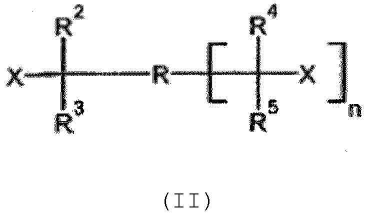 wobei
R² und R³, die voneinander unabhängig sind, ein Wasserstoffatom oder eine C₁₋₆ Alkylgruppe darstellen,
R⁴ und R⁵, die gleich oder verschieden sein können und die voneinander unabhängig sind, ein Wasserstoffatom oder eine C₁₋₆ Alkylgruppe darstellen,
X, das gleich oder verschieden sein kann, eine Gruppe darstellt, die aus einer Carbonsäurehalogenidgruppe, einer Isocyanatgruppe und einer Abgangsgruppe ausgewählt ist,
R eine (n+1)-valente organische Gruppe ist, und n eine ganze Zahl von 1 bis 3 ist; oder
(c2) durch Reagieren von Kohlendioxid mit einem Epoxidharz der folgenden Formel (III): 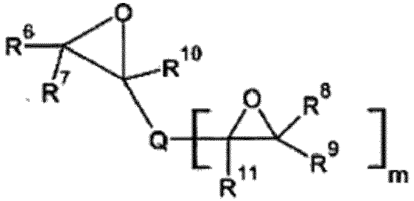 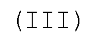 wobei
Q eine (m+1)-valente organische Gruppe ist,
R⁶ und R⁷, die voneinander unabhängig sind, ein Wasserstoffatom oder eine C₁₋₆ Alkylgruppe darstellen,
R⁸ und R⁹, die gleich oder verschieden sein können und die voneinander unabhängig sind, ein Wasserstoffatom oder eine C₁₋₆ Alkylgruppe darstellen,
m eine ganze Zahl von 1 bis 3 ist; und/oder
(c3) Reagieren von Phosgen mit einem Polyol der folgenden Formel (IV) 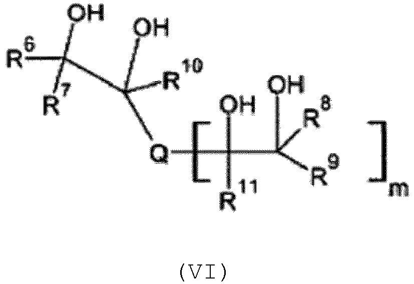 wobei Q, R⁶, R⁷, R⁸, R⁹, R¹⁰, R¹¹ und m unabhängig sind, wie für Formel (III) definiert, zur Herstellung einer Dentalzusammensetzung, die aus einer Wurzelkanalfüllungszusammensetzung und einer Zusammensetzung zur Pulpaüberkappung ausgewählt ist.

## Revendications

1. Composition dentaire choisie parmi une composition d'obturation de canal radiculaire et une composition de coiffage pulpaire, comprenant :
(a) une charge particulaire radio-opaque ;
(b) un monomère ayant au moins deux groupes choisis parmi des groupes amino primaires et secondaires et des groupes thiol ; et
(c) un composé polymérisable avec le monomère (b) dans une réaction de polymérisation par étapes sur la base d'au moins deux groupes choisis parmi les formules (A) et (B) suivantes, attachés à un ou plusieurs groupes organiques ou reliés par ceux-ci : 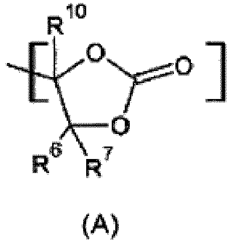 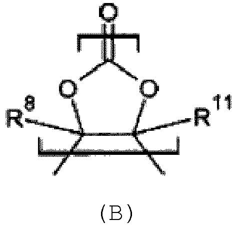 dans lesquelles R⁶, R⁷ R⁸, R¹⁰ et R¹¹, qui sont indépendants les uns des autres, représentent un atome d'hydrogène ou un groupe alkyle en C₁₋₆.

2. Composition dentaire selon la revendication 1, dans laquelle le composé polymérisable avec le monomère (b) dans une réaction de polymérisation par étapes est un composé pouvant être obtenu par
(c1) la réaction d'un mélange comprenant
(i) (n+1) moles d'un ou plusieurs composés de formule (I) suivante : 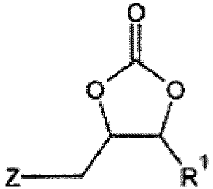 dans laquelle
Z est un groupe hydroxyle, un groupe thiol ou un groupe -NHR°, où R° est un groupe alkyle en C₁₋₆,
R¹ est un atome d'hydrogène ou un groupe alkyle en C₁₋₆,
(ii) par mole d'un composé de formule (II) suivante : 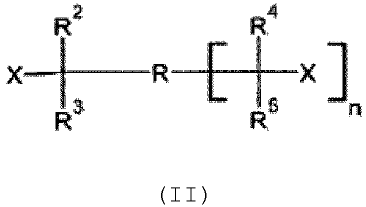 dans laquelle
R² et R³ qui sont indépendants l'un de l'autre, représentent un atome d'hydrogène ou un groupe alkyle en C₁₋₆,
les R⁴ et R⁵, qui peuvent être identiques ou différents et qui sont indépendants les uns des autres, représentent un atome d'hydrogène ou un groupe alkyle en C₁₋₆, ou
dans laquelle deux groupes choisis parmi R², R³ et les R⁴ et R⁵ forment ensemble un groupe alkylène en C₁₋₁₂ ou alcénylène en C₁₋₁₂, respectivement ;
les X, qui peuvent être identiques ou différents, représentent un groupe choisi parmi un groupe halogénure d'acide carboxylique, un groupe isocyanate et un groupe partant,
R est un groupe organique (n+1)-valent, et
n est un entier de 1 à 3,
pour former un composé ayant des groupes 2,3-carbonatopropyle éventuellement substitués par R¹, qui sont polymérisables avec le monomère (b) dans une réaction de polymérisation par étapes ; et/ou
(c2) la réaction de dioxyde de carbone avec une résine époxy de formule (III) suivante : 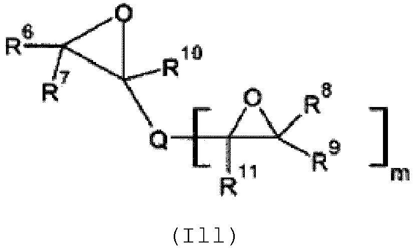 dans laquelle
Q est un groupe organique (m+1)-valent,
R⁶ et R⁷ qui sont indépendants l'un de l'autre, représentent un atome d'hydrogène ou un groupe alkyle en C₁₋₆,
les R⁸ et R⁹, qui peuvent être identiques ou différents et qui sont indépendants les uns des autres, représentent un atome d'hydrogène ou un groupe alkyle en C₁₋₆, ou
dans laquelle deux groupes choisis parmi R⁶, R⁷, R¹⁰ et les R⁸, R⁹ et R¹¹ forment ensemble un groupe alkylène en C₁₋₁₂ ou un groupe alcénylène en C₁₋₁₂,
R¹⁰ et R¹¹ qui sont indépendants l'un de l'autre, représentent un atome d'hydrogène ou un groupe alkyle en C₁₋₆, et
m est un entier de 1 à 3 ; et/ou
(c3) la réaction de phosgène avec un polyol de formule (IV) suivante 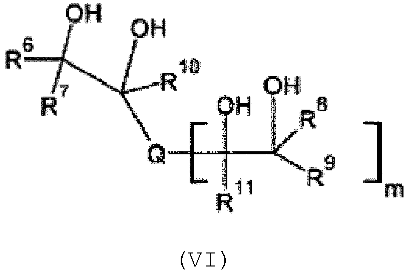 dans laquelle Q, R⁶, R⁷, les R⁸, les R⁹, R¹⁰, les R¹¹ et m sont indépendamment tels que définis pour la formule (III).

3. Composition dentaire selon la revendication 2, dans laquelle R est un groupe hydrocarboné (n+1)-valent ayant 2 à 20 atomes de carbone qui peuvent contenir de 1 à 6 hétéroatomes et/ou groupes choisis parmi des atomes d'oxygène, des atomes de soufre, des groupes amino tertiaires ou des groupes amide, moyennant quoi les hétéroatomes et peuvent former une partie d'une chaîne carbone ou d'un groupe fonctionnel choisi parmi des groupes hydroxyle, des groupes thiol, des groupes carbonyle ou des groupes thiocarbonyle.

4. Composition dentaire selon la revendication 2, dans laquelle Q est un groupe hydrocarboné (m+1)-valent ayant 2 à 20 atomes de carbone qui peut contenir de 1 à 6 hétéroatomes et/ou groupes choisis parmi des atomes d'oxygène, des atomes de soufre, des groupes amino tertiaires ou des groupes amide, moyennant quoi les hétéroatomes et peuvent former une partie d'une chaîne carbone ou d'un groupe fonctionnel choisi parmi des groupes hydroxyle, des groupes thiol, des groupes carbonyle ou des groupes thiocarbonyle.

5. Composition dentaire selon la revendication 3 ou 4, dans laquelle R est un groupe hydrocarboné aliphatique, alicyclique ou aromatique (n+1)-valent qui peut contenir de 1 à 6 hétéroatomes choisis parmi des atomes d'oxygène et des atomes de soufre, ou Q est un groupe hydrocarboné aliphatique, alicyclique ou aromatique (m+1)-valent qui peut contenir de 1 à 6 hétéroatomes et/ou groupes choisis parmi des atomes d'oxygène, des atomes de soufre, des groupes amino tertiaires ou des groupes amide, moyennant quoi les hétéroatomes et peuvent former une partie d'une chaîne carbone ou d'un groupe fonctionnel choisi parmi des groupes hydroxyle, des groupes thiol, des groupes carbonyle ou des groupes thiocarbonyle.

6. Composition dentaire selon la revendication 2, dans laquelle R ou Q est un groupe hydrocarboné aliphatique à chaîne droite ou ramifié ayant 2 à 20 atomes de carbone et qui peut contenir de 1 à 6 hétéroatomes et/ou groupes choisis parmi des atomes d'oxygène, des atomes de soufre, des groupes amino tertiaires ou des groupes amide, moyennant quoi les hétéroatomes et peuvent former une partie d'une chaîne carbone ou d'un groupe fonctionnel choisi parmi des groupes hydroxyle, des groupes thiol, des groupes carbonyle ou des groupes thiocarbonyle.

7. Composition dentaire selon l'une quelconque des revendications précédentes, dans laquelle R ou Q est un groupe aromatique.

8. Composition dentaire selon l'une quelconque des revendications précédentes, dans laquelle R¹ ou chaque R⁶ à R⁹ est un atome d'hydrogène.

9. Composition dentaire selon l'une quelconque des revendications précédentes, dans laquelle R² et R³ qui sont indépendants l'un de l'autre, représentent un atome d'hydrogène ou un groupe méthyle.

10. Composition dentaire selon l'une quelconque des revendications précédentes, dans laquelle les R⁴ et R⁵, qui peuvent être identiques ou différents et qui sont indépendants les uns des autres, représentent un atome d'hydrogène ou un groupe méthyle, ou
dans laquelle n ou m valent 1.

11. Composition dentaire selon l'une quelconque des revendications précédentes, dans laquelle le composé polymérisable par étapes avec le monomère ayant au moins deux groupes choisis parmi des groupes amino primaires et secondaires et des groupes thiol est un composé de formule (V) suivante : 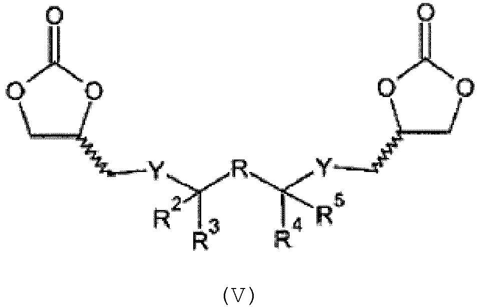 dans laquelle R est tel que défini dans la revendication 1 et R², R³, R⁴ et R⁵ sont indépendants les uns des autres et représentent un atome d'hydrogène ou un groupe méthyle et les Y, qui sont indépendants les uns des autres représentent une liaison, un atome d'oxygène, un atome de soufre, une liaison ester, une liaison uréthane, de préférence dans laquelle R est tel que défini dans l'une quelconque des revendications 2 à 5.

12. Procédé pour la préparation d'un composé polymérisable avec un monomère ayant au moins deux groupes choisis parmi des groupes amino primaires et secondaires et des groupes thiol dans une réaction de polymérisation par étapes sur la base d'au moins deux groupes choisis parmi les formules (A) et (B) suivantes attachés à un ou plusieurs groupes organiques ou reliés par ceux-ci : 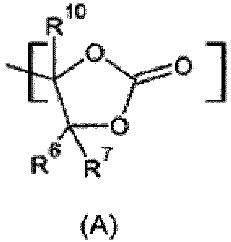 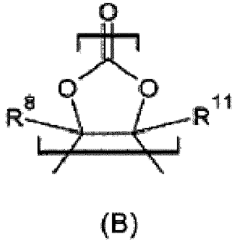 R⁶, R⁷ R⁸, R¹⁰ et R¹¹ qui sont indépendants les uns des autres, représentent un atome d'hydrogène ou un groupe alkyle en C₁₋₆
le procédé comprenant
(c1) la réaction d'un mélange comprenant
(i) au moins (n+1) moles d'un ou plusieurs composés de formule (I) suivante : 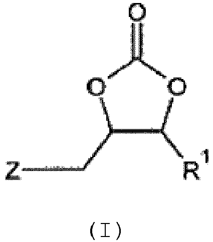 dans laquelle
Z est un groupe hydroxyle, un groupe thiol ou un groupe -NHR^{∘}, où R^{∘} est un groupe alkyle en C₁₋₆,
R¹ est un atome d'hydrogène ou un groupe alkyle en C₁₋₆, et
(ii) une mole d'un composé de formule (II) suivante : 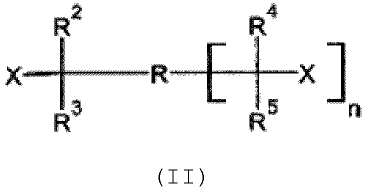 dans laquelle
R² et R³ qui sont indépendants l'un de l'autre, représentent un atome d'hydrogène ou un groupe alkyle en C₁₋₆,
les R⁴ et R⁵, qui peuvent être identiques ou différents et qui sont indépendants les uns des autres, représentent un atome d'hydrogène ou un groupe alkyle en C₁₋₆,
les X, qui peuvent être identiques ou différents, représentent un groupe choisi parmi un groupe halogénure d'acide carboxylique, un groupe isocyanate et un groupe partant,
R est un groupe organique (n+1)-valent, et
n est un entier de 1 à 3 ;
(c2) la réaction de dioxyde de carbone avec une résine époxy de formule (III) suivante : 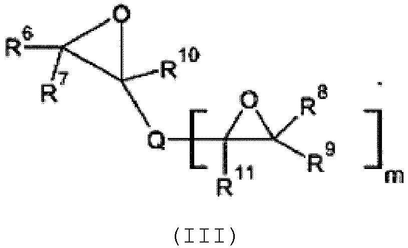 dans laquelle
Q est un groupe organique (m+1)-valent,
R⁶ et R⁷ qui sont indépendants l'un de l'autre, représentent un atome d'hydrogène ou un groupe alkyle en C₁₋₆,
les R⁸ et R⁹, qui peuvent être identiques ou différents et qui sont indépendants les uns des autres, représentent un atome d'hydrogène ou un groupe alkyle en C₁₋₆,
m est un entier de 1 à 3 ; et/ou
(c3) la réaction de phosgène avec un polyol de formule (IV) suivante 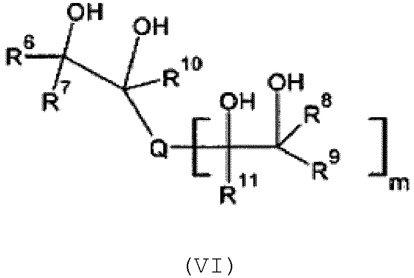 dans laquelle Q, R⁶, R⁷, les R⁸, les R⁹, R¹⁰, les R¹¹ et m sont indépendamment tels que définis pour la formule (III).

13. Utilisation du composé polymérisable avec un monomère ayant au moins deux groupes choisis parmi des groupes amino primaires et secondaires et des groupes thiol dans une réaction de polymérisation par étapes sur la base d'au moins deux groupes choisis parmi les formules (A) et (B) suivantes attachés à un ou plusieurs groupes organiques ou reliés par ceux-ci : 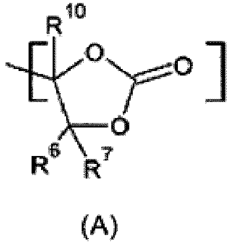 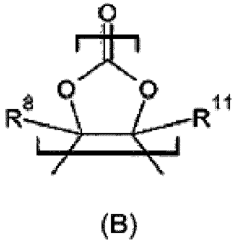 R⁶, R⁷ R⁸, R¹⁰ et R¹¹ qui sont indépendants les uns des autres, représentent un atome d'hydrogène ou un groupe alkyle en C₁₋₆
le composé pouvant être obtenu par
(c1) la réaction d'un mélange comprenant
(i) au moins (n+1) moles d'un ou plusieurs composés de formule (I) suivante : 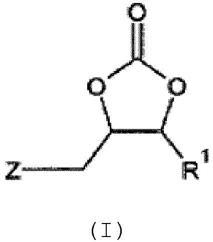 dans laquelle
Z est un groupe hydroxyle, un groupe thiol ou un groupe -NHR^{∘}, où R^{∘} est un groupe alkyle en C₁₋₆,
R¹ est un atome d'hydrogène ou un groupe alkyle en C₁₋₆,
(ii) une mole d'un composé de formule (II) suivante : 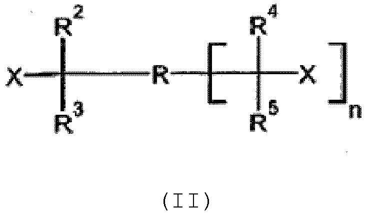 dans laquelle
R² et R³ qui sont indépendants l'un de l'autre, représentent un atome d'hydrogène ou un groupe alkyle en C₁₋₆,
les R⁴ et R⁵, qui peuvent être identiques ou différents et qui sont indépendants les uns des autres, représentent un atome d'hydrogène ou un groupe alkyle en C₁₋₆,
les X, qui peuvent être identiques ou différents, représentent un groupe choisi parmi un groupe halogénure d'acide carboxylique, un groupe isocyanate et un groupe partant,
R est un groupe organique (n+1)-valent, et
n est un entier de 1 à 3 ; ou
(c2) par la réaction de dioxyde de carbone avec une résine époxy de formule (III) suivante : 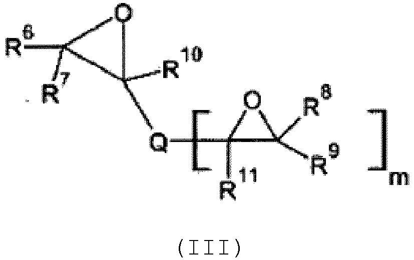 dans laquelle
Q est un groupe organique (m+1)-valent,
R⁶ et R⁷ qui sont indépendants l'un de l'autre, représentent un atome d'hydrogène ou un groupe alkyle en C₁₋₆,
les R⁸ et R⁹, qui peuvent être identiques ou différents et qui sont indépendants les uns des autres, représentent un atome d'hydrogène ou un groupe alkyle en C₁₋₆,
m est un entier de 1 à 3 ; et/ou
(c3) la réaction de phosgène avec un polyol de formule (IV) suivante 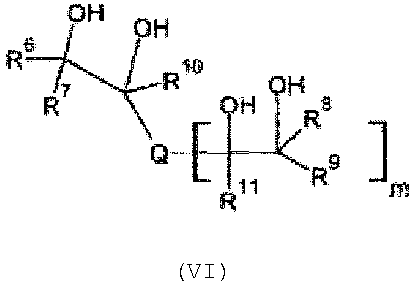 dans laquelle Q, R⁶, R⁷, les R⁸, les R⁹, R¹⁰, les R¹¹ et m sont indépendamment tels que définis pour la formule (lll), pour la préparation d'une composition dentaire choisie parmi une composition d'obturation de canal radiculaire et une composition de coiffage pulpaire.
